# EUROPÄISCHE PATENTSCHRIFT

(11) **EP 1 642 551 B1**
(45) Veröffentlichungstag und Bekanntmachung des Hinweises auf die Patenterteilung: **17.12.2008**
(21) Anmeldenummer: 05019390.3
(22) Anmeldetag: 07.09.2005
(51) Int. Cl.: A61F 2/86

(54) **Implantat mit geringer Radialfestigkeit**
Stent having low radial strength
Stent à faible résistance radiale

(30) Priorität: 09.09.2004 DE 102004044679
(43) Veröffentlichungstag der Anmeldung: 05.04.2006
(73) Patentinhaber: Biotronik VI Patent AG, 6340 Baar (CH)
(72) Erfinder: Gerold, Bodo, Dr., 91225 Zellingen (DE); Harder, Claus, Dr., 91080 Uttenreuth (DE); Müller, Heinz, Dr., 91054 Erlangen (DE); Heublein, Bernd, verstorben (DE)
(74) Vertreter: Lindner-Vogt, Karin L.

(56) Entgegenhaltungen:
- WO-A-20/04043474
- W. SCHMIDT, P. BEHRENS, D. BEHREND, K.-P. SCHMITZ: "Measurement of Mechanical Properties of Coronary Stents according to the European Standard prEN 12006-3" PROGRESS IN BIOMEDICAL RESEARCH, Bd. 4, 28. Februar 1999 (1999-02-28), Seiten 45-51, XP002356848
- VAN DER GIESSEN WJ: "Development of a Polymer Endovascular Prosthesis and its Implantation in Porcine Arteries" JOURNAL OF INTERVENTIONAL CARDIOLOGY, Bd. 5, Nr. 3, 30. September 1992 (1992-09-30), Seiten 175-185, XP009089020

## Beschreibung

Die Erfindung betrifft ein endovaskuläres Implantat bestehend aus einem an den Stirnseiten offenen, rohrförmigen Grundkörper, der von einem nicht expandierten Zustand in einen expandierten Zustand dilatierbar ist.

Seit vielen Jahren hat sich in der Medizintechnik die Implantation von endovaskulären Stützsystemen als eine der wirkungsvollsten therapeutischen Maßnahmen bei der Behandlung von Gefäßerkrankungen etabliert. So hat z. B. bei der interventionellen Therapie der stabilen und instabilen angina pectoris die Einführung von Stents zu einer deutlichen Reduktion der Rate an Restenosen und damit zu besseren Langzeitresultaten geführt. Ursächlich für den Nutzen der Stentimplantation bei vorgenannter Indikation ist der höhere primäre Lumengewinn. Durch den Einsatz von Stents kann zwar ein für den Therapieerfolg primär notwendiger optimaler Gefäßquerschnitt erreicht werden, allerdings injiziert die dauerhafte Anwesenheit eines derartigen Fremdkörpers eine Kaskade von mikrobiologischen Prozessen, die zu einem allmählichen Zuwachsen des Stents führen können. Ein Ansatzpunkt zur Lösung dieser Problematik besteht daher darin, den Stent aus einem biodegradierbaren Werkstoff zu fertigen.

Zur Realisation derartiger biodegradierbarer Implantate stehen dem Medizintechniker verschiedenartigste Werkstoffe zur Verfügung. Neben zahlreichen Polymeren, die häufig zur besseren Biokompatibilität natürlichen Ursprungs oder zumindest an natürliche Verbindungen angelehnt sind, werden in jüngster Zeit metallische Werkstoffe, mit ihren für die Implantate wesentlich günstigeren mechanischen Eigenschaften favorisiert. Besondere Beachtung finden in diesem Zusammenhang magnesium-, eisen- und wolframhaltige Werkstoffe.

Die endovaskulären Implantate werden üblicherweise mit geeigneten Kathetersystemen am Implantationsort dilatiert. Generell lässt sich dabei eine Unterscheidung treffen zwischen selbstexpandierenden Implantaten und Implantaten, die erst durch Inflation eines Ballons des Kathetersystems dilatiert werden. Selbstexpandierende Implantate lassen sich beispielsweise aus Gedächtniswerkstoffen wie Nitinol formen oder sie weisen Strukturelemente auf, deren Zusammenspiel auf mechanischem Wege zur gewünschten Expansion des Implantats führt. Bei der Ballondilatation werden die auf den Ballon des Kathetersystems gecrimpten Implantate gleichmäßig durch den Ballon aufgeweitet bzw. verformt.

Ein Problem bei der Dilatation von endovaskulären Implantaten besteht darin, dass beim Kontakt mit der Gefäßwand bzw. beim Aufweiten des Gefäßes kleinste Verletzungen, Einrisse, Dissektionen der Gefäßwand entstehen, die zwar zumeist problemlos verheilen, jedoch durch das ausgelöste Zellwachstum zu Wucherungen führen können. Dieser negative Effekt wird jedoch in Kauf genommen, um ein übermäßiges Zusammenziehen des Blutgefäßes nach Dilatation mit einhergehender Gefäßquerschnittsverengung (Obstruktion) zu verhindern. Um den Effekt entgegenzuwirken, sehen bisherige Lösungen zum Beispiel vor, Beschichtungssysteme auf das Implantat aufzubringen, die pharmazeutische Substanzen enthalten oder selbst eine derartige Wirkung entfalten. Die bisherigen Systeme sind dabei im wesentlichen auf die Erfordernisse bei der Behandlung stark verengter oder verschlossener Blutgefäße mit atherosklerotischer Plaque ausgelegt. Derartige pathologische Gewebsveränderungen erfordern eine hohe Radialfestigkeit des Implantats, um der Obstruktion des Gefäßes vorzubeugen. Zwar ist bekannt, dass Gefäßverletzungen zu einer inflammatorischen Antwort des Gewebes führen und letztendlich die neointimale Hyperplasie fördern (R. Kornowski, M. K. Hong, F. O. Tio, O. Bramwell, H. Wu, M. B. Leon, JACC, Vol. 31 (1998), Nr. 1, 224-230), jedoch wurden die geschilderten Nachteile mangels Alternativen bisher in Kauf genommen.

In US 5,888,201 wird angesprochen, dass Stents mit einer moderaten Steifigkeit und einem relativ geringen Elastizitätsmodul einem breiten Anwendungsbereich zugänglich wären. Es wird eine selbstexpandierende Stentstruktur aus einer biokompatiblen, aber nicht biodegradierbaren Titanlegierung beschrieben.

Neben den mechanischen Einflüssen auf die Gefäßwand bei der Dilatation stellt auch die dauerhafte Anwesenheit der Implantate herkömmlicher Bauweise einen Angriffspunkt der Restenose dar. Häufig ist eine dauerhafte Anwesenheit des Implantats jedoch aus medizinischer Sicht nicht notwendig. Daher bestehen Bestrebungen das Implantat aus einem biodegradierbaren Werkstoff zu formen. Bekannt sind in diesem Zusammenhang biodegradierbare Metalllegierungen, z.B. auf Basis von Magnesium, Eisen, Zink oder Wolfram.

WO 2004/043474 wird als nächstligender Stand der Technik gegenüber dem Gegenstand des Anspruchs 1 angesehen.

Eine Aufgabe der vorliegenden Erfindung war es, ein endovaskuläres Implantat zur Verfügung zu stellen, das den pathologischen Gegebenheiten einer Vielzahl von Gefäßerkrankungen außerhalb der atherosklerotischen Plaque besser angepasst ist.

Diese Aufgabe wird durch das endovaskuläre Implantat nach Anspruch 1 gelöst. Das endovaskuläre Implantat aus einem biodegradierbaren Werkstoff besitzt einen an den Stirnseiten offenen, rohrförmigen Grundkörper, der von einem nicht expandierten Zustand in einen expandierten Zustand dilatierbar ist. Das Implantat ist derart ausgelegt, dass bei Beaufschlagung des Implantats im expandierten Zustand mit einem radial wirkenden Kompressionsdruck im Bereich von 5 bis 30 kPa (0,05-0,3 bar)
- eine Querschnittsfläche des Implantats auf 70 % oder weniger der ursprünglichen Querschnittsfläche verringert wird oder
- ein Innenvolumen des Implantats auf 70 % oder weniger des ursprünglichen Innenvolumens verringert wird.

Mit anderen Worten, die erfindungsgemäßen endovaskulären Implantate zeichnen sich u.a. durch eine geringere Radialfestigkeit als die bisher bekannten, gattungsgemäßen Implantate des Standes der Technik aus. Für viele Indikationen, die bisher nur mit herkömmlichen Implantaten behandelt wurden, kann nun mit Hilfe des erfindungsgemäßen Implantats der Heilungsverlauf wesentlich verbessert werden.

Überraschenderweise hat die Anmelderin bei histologischen Studien festgestellt, dass eine geringere Radialfestigkeit von biodegradierbaren Implantaten für eine Vielzahl von pathologischen Gefäßveränderungen nicht nur tolerabel ist, sondern zu einer deutlichen Verbesserung des Heilungsprozesses führt. Bislang stand allein das mechanische Offenhalten eines stenosierten Gefäßes im Vordergrund, wobei ein und derselbe Stenttyp für pathophysiologisch durchaus unterschiedliche Läsionen eingesetzt wurde. Ziel bei der Entwicklung herkömmlicher biodegradierbarer Stents war es daher stets, eine möglichst hohe Radialfestigkeit mit möglichst geringem Recoil zu erzielen. Offenbar ist aber eine sehr hohe Radialfestigkeit des Stents nicht für alle Läsionen vorteilhaft, sondern kann aufgrund der damit verbundenen hohen mechanischen Belastung der Gefäßwand und der dadurch induzierten Proliferation glatter Gefäßmuskelzellen sogar nachteilig sein. Aufgrund der Komplexität der Gefäßveränderungen scheint ein biodegradierbares Implantat mit geringerer Radialfestigkeit gerade im Bereich vulnerabler Plaques sowie bei der Behandlung von Dissektionen der Gefäßwand besonders vorteilhaft zu sein. Bei übermäßiger mechanischer Beanspruchung vulnerabler Plaques kann durch Austreten lipider Partikel ein Thrombus in der Blutbahn entstehen, der Ausgangspunkt akuter Infarkte sein kann. Selbst bei nur geringer Radialfestigkeit kann die dauerhafte Anwesenheit des Implantats Ausgangspunkt für entzündliche oder immunologische Prozesse sein, die den Heilungsprozess behindern. Durch den allmählichen Abbau des Implantats im Körper wird einer solchen negativen Entwicklung der Boden entzogen.

Unter "Radialfestigkeit" wird ein innerer Widerstand des Implantats in seinem expandierten Zustand gegen radial wirkende Kräfte verstanden, die eine radiale Kompression des Implantats bewirken könnten. Die Radialfestigkeit lässt sich quantitativ durch Angabe eines Kollapsdruckes ausdrücken. Die Implantate des Standes der Technik zeigen dabei ein Kollapsverhalten, bei dem sich die Kompression schlagartig vollzieht, d.h. mit Erreichen des Kollapsdruckes kollabiert das Implantat sehr rasch. In vivo ist dieses Kollapsverhalten jedoch unerheblich, da alle gängigen Implantate ausgelegt sind, den im Körper auftretenden Kräften zu widerstehen. Das durch die Zielsetzung einer möglichst hohen Radialfestigkeit bedingte Kollapsverhalten der Implantate des Standes der Technik ist jedoch für die Zwecke der vorliegenden Erfindung nicht zwingend erforderlich bzw. stellt sich bei den erfindungsgemäßen Implantaten nicht zwangsläufig ein. Zu Zwecken der Klarstellung wurden daher die Begriffe "Kompressionsdruck" und "Kompressionsverhalten" eingeführt, die dem flexibleren Verhalten auf radial wirkende Drücke Ausdruck verleihen sollen. So ist beispielsweise denkbar, dass die Querschnittsfläche bzw. das Innenvolumen eine monoton fallende Funktion des radial angelegten Druckes ist, wobei sich bei Erreichen des Kompressionsdruckes die erfindungsgemäße Verringerung der Querschnittfläche oder des Innenvolumens einstellt. Mit anderen Worten, ausgehend vom expandierten Zustand verringert sich mit steigendem radial angelegten Druck allmählich die Querschnittsfläche bzw. das Innenvolumen zunehmend bis der Kompressionsdruck erreicht wird. Es ist auch denkbar, dass die Funktion der Querschnittsfläche bzw. des Innenvolumens nur bis zu einem vorgebbaren Grenzdruck oder Grenzdurchmesser oder Grenzquerschnitt monoton fallend verläuft und sich danach abflacht (nichtlinearer Druck-Durchmesser-Verlauf). So kann ein weitgehender Verschluss des Gefäßes/ des Implantates bei gleich bleibender Beaufschlagung mit einem Kompressionsdruck vermieden werden.

Unter "Kompressionsdruck" im erfindungsgemäßem Sinne wird der experimentell bestimmte Druckwert verstanden, bei dessen Beaufschlagung das in einem Prüfstand eingespannte expandierte Implantat 70 % des expandierten (ursprünglichen) Innenvolumens unterschreitet. Alternativ kann der Kompressionsdruck definiert werden, als der Druck, bei dem das expandierte Implantat an einem vorgegebenen Querschnitt durch das Implantat 70 % der expandierten (ursprünglichen) Querschnittsfläche unterschreitet. Dazu wird - ganz analog zur bekannten Bestimmung des Kollapsdruckes herkömmlicher Implantate - das zu prüfende Implantat im expandierten Zustand von einem dünnwandigen Polyurethan-Schlauch (Wandstärke 0,075 mm) aufgenommen, dessen unteres Ende verschlossen ist und dessen oberes Ende über ein Rohr gestülpt ist, das einen atmosphärischen Druckausgleich mit dem Innenraum des Schlauchs ermöglicht. Das von dem Schlauch umhüllte Implantat wird anschließend und in eine mit 37°C warmen Wasser befüllte, verschließbare Prüfkammer eingeführt. Mit Hilfe einer angeschlossenen Drucksteuerung wird die Prüfkammer in 5 bis 10 kPa-Schritten (0,05 bis 0,1bar-Schritten) mit Druck beaufschlagt. Über einen Lasermesskopf, dessen Messstrahl z.B. etwa auf die Implantatsmitte gerichtet ist, wird die Verformung des Implantats, d.h. eine Verringerung der Querschnittsfläche infolge der Druckbeaufschlagung gemessen. Bei gleichmäßiger Kompression des Implantats kann aus der erfassten Querschnittsflächenänderung direkt auch eine Innenvolumenänderung abgeleitet werden. Kollabiert das Implantat jedoch bei Druckbeaufschlagung über seine Länge ungleichmäßig, so wird die Messung an mehreren Punkten des Implantats (gleichzeitig oder in mehreren Versuchsreihen) durchgeführt. Mit einem 2-Achsen-Laserscanner können bei jeder Druckstufe die Querschnittsveränderungen an verschiedenen Stellen des Implantats gleichzeitig über seine ganze Länge erfasst werden. Die Anzahl und Lage dieser Messpunkte wird durch den Versuchsleiter vorgegeben, mit der Maßgabe den Vorgang des Komprimierens möglichst exakt über die Länge des Implantats abzubilden. Mit den gewonnen Messwerten kann die Innenvolumenänderung bei Druckbeaufschlagung auch bei ungleichmäßig kollabierenden Implantaten größenmäßig erfasst werden.

Zu weiteren Einzelheiten bei Bestimmung des Kollapsdrucks als Maß für die Radialfestigkeit sei auf die Artikel von
- K.-P. Schmitz, D. Behrend, P. Behrens, W. Schmidt, W. Urbaszek, Experimentelle Überprüfung des Aufweitverhaltens von ballonexpandierbaren Koronarstents als Grundlage für in vitro Untersuchungen, Biomedizinische Technik 42 (1997), 203-204,
- W. Schmidt, D. Behrend, A. Nawroth, K.-P. Schmitz, Experimentelle Bestimmung der Qualitätsparameter von ballonexpandierbaren Koronarstents, Biomedizinische Technik 40 (1995), 363-364 und
- W. Schmidt, D. Behrend, P. Behrens, R. Jung, W. Urbaszek, K.-P. Schmitz, Intravaskuläre Stents - Konstruktionsprinzipien und Aufweitverhalten, Biomedizinische Technik 41 (1996), 400-401
verwiesen, deren Inhalt hiermit vollumfänglich einbezogen wird. Die dort beschriebene Herangehensweise lässt sich ganz analog auf die Bestimmung der erfindungsgemäßen Kompressionsdrücke übertragen.

Der Begriff "biodegradierbarer Werkstoff" bedeutet vorliegend, dass der Werkstoff in vivo abgebaut wird und das Implantat in der Folge seine ursprüngliche medizintechnische Funktionalität nicht mehr wahrnehmen kann. Nicht notwendiger Weise müssen die Abbauprodukte vollständig vom Körper resorbiert oder ausgeschieden werden. Es können beispielsweise auch kleine Partikel am Ort der Applikation verbleiben. Biodegradation im erfindungsgemäßen Sinne betrifft insbesondere hydrolytische, enzymatische und andere stoffwechselbedingte Abbauprozesse im lebendem Organismus, die zu einer allmählichen Auflösung zumindest großer Teile der verwendeten Materialien führen. Synonym wird häufig der Begriff Biokorrosion verwendet. Der Begriff Bioresorption umfasst zusätzlich die anschließende Resorption der Abbauprodukte.

Nach einer bevorzugten Ausführungsform der Erfindung ist das Implantat aus einer Magnesium-, Eisen- oder Wolframlegierung, geformt. Besonders bevorzugt sind Magnesiumlegierungen vom Typ WE, insbesondere WE43. Letztere Legierungen zeichnen sich durch die Anwesenheit von Seltenerden und Yttrium aus. Die genannten Werkstoffe lassen sich einfach verarbeiten, haben geringe Materialkosten und lassen aufgrund ihrer intrinsischen Werkstoffeigenschaften die Umsetzung von strukturellen Maßnahmen zur Erzielung der gewünschten Kollapsdruckbereiche zu. Weiterhin wurde zumindest für einen Teil der Legierungen ein positiver physiologischer Effekt der Abbauprodukte auf den Heilungsprozess festgestellt. Ferner hat sich gezeigt, dass aus WE43 hergestellte Magnesiumstents kein störendes Magnetresonanz-Artefakt erzeugen, wie von medizinischem Edelstahl (316L) bekannt, weshalb das perfundierbare Lumen des Stents zu jedem Zeitpunkt nach Implantation mit MR-Tomographie beurteilt werden kann.

Die biodegradierbare Metalllegierung aus den Elementen Magnesium, Eisen oder Wolfram enthalten die genannten Elemente jeweils in einem Anteil von mindestens 50 Gew.%, insbesondere mindestens 70 Gew.%, besonders bevorzugt mindestens 90 Gew.% an der Legierung.

Die Implantate können das erfindungsgemäße Kompressionsverhalten schon im nicht degradiertem Zustand, d.h. vor Implantation und Einsetzten der korrosiven Prozesse zeigen. Eine bevorzugte Variante sieht jedoch vor, dass das Implantat aus der biodegradierbaren Legierung so beschaffen ist, dass sich die erfindungsgemäße Radialfestigkeit erst 1 bis 120 h, insbesondere 24 bis 72 h, nach Einbringung in 37°C warmes, künstliches Plasma nach EN ISO 10993-15:2000 einstellt. Die Versuchsbedingungen erlauben Rückschlüsse auf das in vivo Verhalten des Implantats. Ein Abbauverhalten des Implantats ist unter anderem abhängig von den Materialeigenschaften (Legierungszusammensetzung, Verarbeitungsweise, Morphologie des Materials, etc.) dem Implantatsdesign (Volumenoberfläche, Aufbau der Strukturelemente, etc.) und Oberflächenmodifikationen (Passivierung, Beschichtungen aus biodegradierbaren Polymeren, Rauhigkeit, etc.) und muss in der Regel experimentell bestimmt werden.

Eine weitere, bevorzugte Ausführungsform des erfindungsgemäßen Implantats sieht vor, dass das Implantat aus einem Werkstoff mit einem E-Modul zwischen 10 bis 60 GPa, insbesondere zwischen 40 bis 50 GPa, geformt ist. Ein Vorteil von Werkstoffen mit einem E-Modul in den genannten Bereichen zeigt sich darin, dass zur Erzielung einer erfindungsgemäß niedrigen Radialfestigkeit eine Breite der Stege, die Bestandteil des Stützgerüst des Implantats sind, nicht oder nur in einem gegenüber gängigen, in der Implantatstechnik eingesetzten Werkstoffen geringerem Umfang reduziert werden muss. Ein ausreichend breiter Steg kann erwünscht sein, um die Flächenpressung zwischen Steg und Gefäßwand niedrig zu halten. Eine zu hohe Flächenpressung kann zu Verletzungen an der Gefäßwand führen. Diese Eigenschaft erlangt insbesondere bei vulnerabler Plaque Bedeutung. Weiterhin erleichtern und verbessern höhere Stegbreiten die Herstellung und das Freisetzungsverhalten von LDD-Systemen (LDD = lokal drug delivery), die eine Wirkstoffelution aus der Oberfläche eines Steges erlauben, z.B. durch Aufbringung des Wirkstoffs oder einer den Wirkstoff enthaltenden Beschichtung auf der Stegoberfläche.

Erfindungsgemäße endovaskuläre Implantate eignen sich zur intravaskulären Behandlung vulnerabler Plaque ("Plaque Sealing") oder zur Abdeckung einer Dissektion eines vaskulären Gefäßes. Daher wird die Verwendung der Implantate im Zusammenhang mit diesen Indikationen gesondert hervorgehoben.

Bei beiden genannten Indikationen wird insbesondere von dem besonderen Vorteil der Kombination aus geringer Radialfestigkeit und Abbaubarkeit durch Einsatz eines biodegradierbaren Werkstoffs Gebrauch gemacht:
- Im Vordergrund steht nicht das mechanische Offenhalten des Gefäßes, sondern eine nur temporär erforderliche Stabilisierung der Gefäßwand. Im Fall vulnerabler Plaque möchte man die Gefahr eine Ruptur durch das Lipid verhindern, die dann nicht mehr gegeben ist, wenn sich die Lipideinlagerung zurückgebildet hat. Im Fall der Behandlung von Dissektionen "heftet" man das Dissekat an die Gefäßwand, das dort wieder anwächst.
- Beide Indikationen erfordern lediglich die temporäre Wirkung eines Implantats, weshalb gerade ein degradierbarer Stent verwendet werden sollte, der vorzugsweise zudem antiproliferative Eigenschaften hat, wie etwa am Beispiel von Stents aus der Magnesiumlegierung WE43 nachgewiesen werden konnte. Herkömmliche permanente Stents haben bekanntermaßen Nachteile bezüglich ihrer Restenoserate (die auch bei hämodynamisch wenig relevanten Läsionen deutlich von Null verschieden ist) sowie ihrer Voraussetzungen für eine möglicherweise später erforderliche Re-Intervention und ihrer diagnostischen Möglichkeiten, etwa bedingt durch MR-Artefakte.

Die Erfindung wird nachfolgend anhand von Ausführungsbeispielen und den dazugehörigen Zeichnungen näher erläutert. Es zeigen:
Fig. 1 eine photographische Wiedergabe eines Stents nach Einlagerung in künstlichem Plasma für 48 h,
Fig. 2 eine Kammer zur Prüfung der Radialfestigkeit,
Fig. 3 ein weiteres Design für einen Stent mit der erfindungsgemäßen Radialfestigkeit,
Fig. 4 ein weiteres Design für einen Stent mit der erfindungsgemäßen Radialfestigkeit und
Fig. 5 ein weiteres Design für einen Stent mit der erfindungsgemäßen Radialfestigkeit.

### Ausführungsbeispiel 1

Untersucht wurden sterilisierte Stents aus der in vivo degradierbaren Magnesiumlegierung WE43. Die Stents wurden für die Untersuchungen fortlaufend nummeriert (Nr.1-14). Die Stents hatten einen Durchmesser von 3,0 mm und die Stents Nr. 1, 3 und 5 eine Länge von 15 mm, während die Stents Nr. 2, 4 und 6 bis 14 eine Länge von 10 mm aufwiesen.

Es wurde die Radialfestigkeit der degradierbaren Stents nach Lagerung in korrosiver Umgebung untersucht. Dazu wurde nach definierter Einlagerungszeit der Kompressionsdruck als Maß für die Radialfestigkeit gemessen.

Vor der Einlagerung in das Prüfmedium wurden die als bare-Stents vorliegenden Prüfobjekte in sterilisierte Polyurethan-Schläuche (Innendurchmesser 3 mm, Wandstärke 0,075 mm, Sterilisation mit Formaldehyddampf (FAD)) mit innen liegender distaler Hülse (Teflon) implantiert. Die Implantation erfolgte manuell unter sterilen Laminarflow-Bedingungen.

Zunächst wurde der Stent dem Probenbehälter entnommen und manuell auf einen sterilen Ballonkatheter (Biotronik Lekton Motion) gecrimpt. Der Stent wurde nun im PU-Schlauch mit Nenndruck 600 kPa (6 bar) aufgeweitet. Dadurch wurde der PU-Schlauch überdehnt, was zu einer statischen Belastung des Stents infolge der Wandspannungen im PU-Schlauch führte. Beim verwendeten Prüfschlauchdurchmesser von Innendurchmesser von 3,0 mm und einer Wandstärke von 0,075 mm führte eine Überdehnung von 0,050 mm zu einer zusätzlichen statischen Belastung von 23 kPa (0,23 bar). Der Ballonkatheter wurde anschließend wieder entfernt. Ein Ballonkatheter wurde für drei Stentaufweitungen verwendet.

Der Prüfschlauch wurde mit einer Einwegspritze und Kanüle mit Prüfmedium gefüllt und anschließend in 50 ml Prüfmedium gelagert, so dass die Stents während der Einlagerung in vollständigem Kontakt zum Prüfmedium waren. Als Prüfmedium kam steriles, künstliches Plasma, wie es nach EN ISO 10993-15:2000 für Degradationsuntersuchungen vorgesehen ist, mit folgender Rezeptur zum Einsatz:
NaCl 6.800 g/l
CaCl₂ 0.200 g/l
KCI 0.400 g/l
MgSO₄ 0.100 g/l
NaHCO₃ 2.200 g/l
Na₂HPO₄ 0.126 g/l
NaH₂PO₄ 0.026 g/l

Insgesamt elf verschlossene Probenbehälter mit je 50 ml Prüfmedium und je einem PU-Schlauch mit Mg-Stent wurden anschließend in einem Brutschrank bei 37°C gelagert.

Die insgesamt 14 untersuchten Mg-Stents wurden in fünf Gruppen untersucht. Dabei diente die erste Gruppe mit 3 Stents (Nr. 10, 11, 12) als Referenz (ohne Prüfmedienkontakt). Je 3 Stents wurden nach 24, 48 und 72 sowie 2 Stents 120 h dem Prüfmedium entnommen und die Radialfestigkeit gemessen.

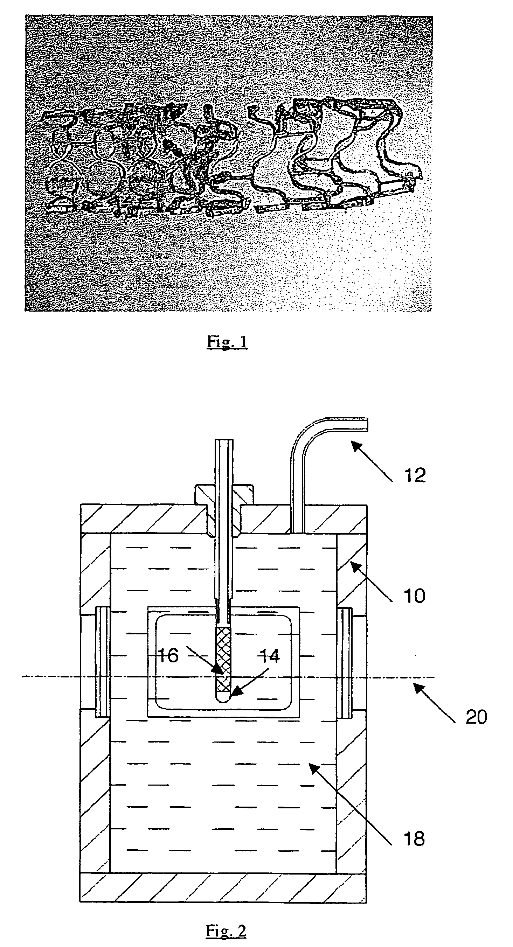

Zur Messung der Radialfestigkeit wurde der sich in einem dünnwandigen PUR-Schlauch 14 (Innendurchmesser 3,0 mm, Wandstärke 0,075 mm) befindliche Stent 16 in einem mit Wasser (37°C) befüllten Prüfbecken 10 gelagert (Fig. 2). Das Prüfmedium wurde aus dem PUR-Schlauch 14 abpipettiert.

Das Prüfbecken 10 wurde druckdicht verschlossen und mit einer Drucksteuerung 12 (BALTUS) verbunden. Der Druck im Becken 18 wurde schrittweise in 5 kPa-Schritten (0,05 bar-Schritten) bis max. 150 kPa (1,5 bar) erhöht und bewirkte über den PUR-Schlauch 14 eine Radialbelastung auf den Stent 16. Dabei wurde in Stentmitte die Durchmesseränderung in Abhängigkeit vom Umgebungsdruck mittels Laser 20 gemessen. Der Kompressionsdruck, bei dem sich die Querschnittsfläche des Stents 16 auf 70 % oder weniger der ursprünglichen Querschnittsfläche verringert, wurde notiert.

Die gemessenen Kompressionsdruckwerte sind für die untersuchten Stents in Abhängigkeit der Einlagerungszeit in der folgenden Tabelle eingetragen. Figur 1 zeigt exemplarisch Stent Nr. 8 nach Entnahme aus dem künstlichen Plasma.

**Tabelle 2: Messergebnisse (1 bar = 100 kPa)**

| Einlagerungszeit [h] | Stent (Nummer) | Kompressionsdruck [bar]* | mittlerer Kompressionsdruck [bar]* |
|---|---|---|---|
| 0 | (10) | 1.05 | 0.85 |
| | (11) | 0.85 | |
| | (12) | 0.65 | |
| 24 | (2) | 0.70 | 0.60 |
| | (4) | 0.55 | |
| | (6) | 0.55 | |
| 48 | (7) | 0.30 | 0.38 |
| | (8) | 0.45 | |
| | (9) | 0.40 | |
| 72 | (1) | 0.45 | 0.30 |
| | (3) | 0.15 | |
| | (5) | 0.30 | |
| 120 | (13) | 0.25 | 0.225 |
| | (14) | 0.20 | |

### Ausführungsbeispiel 2

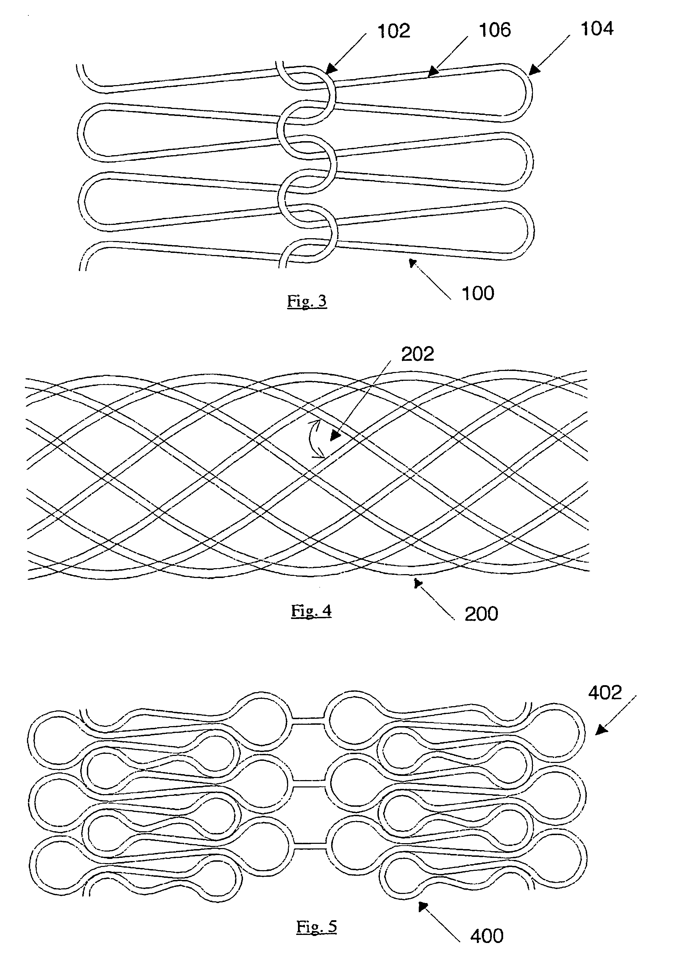

Figur 3 zeigt in einem Ausschnitt aus einer Abwicklung eines Stents eine ballonexpandierbare Struktur 100 auf Drahtbasis, die sich aus einer Vielzahl einzelner Ringsegmente zusammensetzt. Durch die flexible Verbindung 102 der einzelnen Ringsegmente werden auch bei gekrümmten Gefäßen keine Querkräfte übertragen. Der Biegeradius 104 wird an die Werkstoffeigenschaften sowie den gewünschten maximalen Öffnungsdurchmesser angepasst. Die Länge des Elementes 106 bestimmt zusammen mit dem Drahtdurchmesser die Radialfestigkeit. Als Werkstoff eignet sich insbesondere die biodegradierbare Magnesiumlegierung WE43.

### Ausführungsbeispiel 3

Figur 4 zeigt eine selbstexpandierbare Struktur 200 auf Drahtbasis.

Nachfolgend werden verschiedene Maßnahmen am Stentdesign beschrieben, anhand derer die Radialfestigkeit auf den erfindungsgemäßen Wertbereich eingestellt werden kann. Die Maßnahmen fußen auf gefestigte theoretischer Überlegungen und sind dem Fachmann an sich geläufig, so dass deren praktische Umsetzung mit geringem experimentellem Aufwand vollzogen werden kann. Die Maßnahmen sind jeweils aufeinander abzustimmen, damit sich eine Radialfestigkeit im gewünschten Wertbereich einstellt.

Eine erste Maßnahme sieht vor, dass ein biodegradierbarer Werkstoff mit niedrigem E-Modul eingesetzt wird. Beispielhaft bietet sich die Magnesiumlegierung WE43 mit einem E-Modul von ungefähr 45.000 MPa an. Wird die abgebildete Struktur aus zwanzig Drähten mit Drahtdurchmesser 0,09 mm, einem Winkel 202 von 110° zu einem Stent mit Durchmesser von 5 mm gewickelt, so zeigt eine beispielhafte Untersuchung, dass eine Radialfestigkeit von ungefähr 5 kPa (0,05 bar) zu erwarten ist. Diese Untersuchung wird in US 5,888,201 von Stinson et al. näher ausgeführt. Dabei wird der Einfluss der Reibung zwischen Stent und Gefäßwand nicht berücksichtigt. Eine der typischen Eigenschaften dieses Ausführungsbeispiels ist die relativ große Längenänderung bei einer Durchmesseränderung von vorzugsweise 70 % des Nominaldurchmessers. Wird die Längenänderung durch Wandreibung behindert, wird auch die Durchmesseränderung behindert, was zu einer erhöhten Radialfestigkeit führt. Theoretische Überlegungen zeigen, dass sich der Effekt durch eine Reduktion des Winkels 202 reduziert werden kann. Die Einstellung der gewünschten Radialfestigkeit kann demnach auch durch Änderung des Winkels 202 erreicht werden. Theoretische Überlegungen, die all diese Umstände berücksichtigen zeigen, dass sich die Radialfestigkeit eines Stents aus Werkstoff und Dimensionen wie oben beschrieben nach der Implantation von den errechneten 5 kPa (0,05 bar) auf ca. 20 kPa (0,2 bar) erhöhen wird. Sollten Experimente (beispielsweise Messung des Kollapsdruck nach der obig beschriebenen Methode) zeigen, dass die gewünschte Radialfestigkeit von beispielsweise 20 kPa (0,2 bar) nicht erreicht wird, so müsste vorzugsweise der Drahtdurchmesser von 0,09 mm wie oben beschrieben auf beispielsweise 0,1 mm erhöht werden. Sollte die gemessene Radialfestigkeit zu hoch sein, so zeigen theoretische Überlegungen, dass eine Verringerung des Drahtdurchmessers oder auch eine Reduktion des Winkels 202 auf beispielsweise 90°, anstelle der oben erwähnten 110° zu einer Reduktion der Radialfestigkeit führt. Vorzugsweise sollte in diesem Falle der Winkel 202 reduziert werden, da sich durch die Winkelreduktion auch eine reduzierte Längenänderung einstellt und sich somit die Abhängigkeit der Radialfestigkeit von der Wandreibung reduziert. Theoretische Überlegungen lassen eine hohe Variabilität der Wandreibung je nach Stenosentyp erwarten, gemäß obigen Überlegungen wäre bei diesem Ausführungsbeispiel entsprechend auch eine hohe Variabilität der Radialfestigkeit zu erwarten. Durch eine Reduktion des Winkels 202 kann die Varibilität der Radialfestigkeit reduziert werden.

Eine weitere Maßnahme zur Reduktion der Radialfestigkeit kann durch die Reduktion der Anzahl verwobenen Drähte erreicht werden. Die Anzahl der verwobenen Drähte ist nach unten begrenzt, da sich bei einer zu geringen Anzahl Drähte die freien Enden unerwünschterweise nach außen oder innen formen können. Diese Zusammenhänge sind in US 5,061,275 von Wallsten et al. beschrieben.

### Ausführungsbeispiel 4

Figur 5 zeigt eine selbstexpandierbare Struktur 400 (hier in geschlossenem Zustand), die aus einem Magnesiumrohr aus der Legierung WE43 mit einem Nominaldurchmesser von 8 mm geschnitten wird. Durch die großen Radien 402 wird das geringe Dehnvermögen der Magnesiumlegierung nicht überschritten und gleichzeitig die gewünschte Radialfestigkeit vorgegeben.

## Patentansprüche

1. Endovaskuläres Implantat aus einem biodegradierbaren Werkstoff und mit einem an den Stirnseiten offenen, rohrförmigen Grundkörper, der von einem nicht expandierten Zustand in einen expandierten Zustand dilatierbar ist, **dadurch gekennzeichnet, dass** das Implantat derart ausgelegt ist, dass bei Beaufschlagung des Implantats im expandierten Zustand mit einem radial wirkenden Kompressionsdruck im Bereich von 5 bis 30 kPa (0,05 bis 0,3 bar)
- eine Querschnittsfläche des Implantats auf 70% oder weniger der ursprünglichen Querschnittsfläche verringert wird oder
- ein Innenvolumen des Implantats auf 70% oder weniger des ursprünglichen Innenvolumens verringert wird.

2. Implantat nach Anspruch 1, **dadurch gekennzeichnet, dass** der Kompressionsdruck im Bereich von 10 bis 20 kPa (0,1 bis 0,2 bar) liegt.

3. Implantat nach Anspruch 1 oder 2, **dadurch gekennzeichnet, dass** das Implantat aus einem Werkstoff mit einem E-Modul zwischen 10 bis 60 GPa geformt ist.

4. Implantat nach Anspruch 3, **dadurch gekennzeichnet, dass** das Implantat aus einem Werkstoff mit einem E-Modul zwischen 40 bis 50 GPa geformt ist.

5. Implantat nach einem der vorhergehenden Ansprüche, **dadurch gekennzeichnet, dass** der biodegradierbare Werkstoff eine biodegradierbare Magnesium-, Eisen-oder Wolframlegierung ist.

6. Implantat nach Anspruch 5, **dadurch gekennzeichnet, dass** die Magnesiumlegierung eine Legierung vom Typ WE ist.

7. Implantat nach Anspruch 6, **dadurch gekennzeichnet, dass** die Legierung vom Typ WE43 ist.

## Claims

1. An endovascular implant of a biodegradable material and with a tubular base body which is open on the end faces and is dilatable from an unexpanded state into an expanded state,
**characterized in that**
the implant is designed so that when the implant in the expanded state is acted upon by a compression pressure acting radially in the range of 5 kPa to 30 kPa (0.05 to 0.3 bar),
- a cross-sectional area of the implant is reduced to 70% or less of the original cross-sectional area or
- an internal volume of the implant is reduced to 70% or less of the original internal volume.

2. The implant according to Claim 1, **characterized in that** the compression pressure is in the range of 10 kPa to 20 kPa (0.1 to 0.2 bar).

3. The implant according to Claim 1 or 2, **characterized in that** the implant is shaped from a material having a modulus of elasticity between 10 GPa and 60 GPa.

4. The implant according to Claim 3, **characterized in that** the implant is shaped from a material having a modulus of elasticity between 40 GPa and 50 GPa.

5. The implant according to any one of the preceding claims, **characterized in that** the biodegradable material is a biodegradable magnesium alloy, iron alloy or tungsten alloy.

6. The implant according to Claim 5, **characterized in that** the magnesium alloy is an alloy of the WE type.

7. The implant according to Claim 6, **characterized in that** the alloy is of the WE43 type.

## Revendications

1. Implant endovasculaire à base d'un matériau biodégradable et comprenant un corps de base tubulaire, ouvert sur les côtés avant, qui peut être dilaté à partir d'un état non expansé dans un état expansé, **caractérisé en ce que** l'implant est conçu de telle sorte que, en cas de sollicitation de l'implant dans l'état expansé avec une pression de compression agissant radialement dans la plage de 5 à 30 kPa (0.05 à 0.3 bar)
- une surface de section de l'implant est réduite à 70% ou moins de la surface de section initiale ou
- un volume intérieur de l'implant est réduit à 70% ou moins du volume intérieur initial.

2. Implant selon la revendication 1, **caractérisé en ce que** la pression de compression se situe dans la plage de 10 à 20 kPa (0.1 à 0.2 bar).

3. Implant selon la revendication 1 ou 2, **caractérisé en ce que** l'implant est formé à partir d'un matériau présentant un module d'élasticité compris entre 10 et 60 GPa.

4. Implant selon la revendication 3, **caractérisé en ce que** l'implant est formé à partir d'un matériau présentant un module d'élasticité compris entre 40 et 50 GPa.

5. Implant selon l'une quelconque des revendications précédentes, **caractérisé en ce que** le matériau biodégradable est un alliage biodégradable de magnésium, de fer ou de tungstène.

6. Implant selon la revendication 5, **caractérisé en ce que** l'alliage de magnésium est un alliage de type WE.

7. Implant selon la revendication 6, **caractérisé en ce que** l'alliage est du type WE43.
